(19) Europäisches Patentamt
European Patent Office
Office européen des brevets

(11) **EP 3 925 520 A1**

(12) **EUROPEAN PATENT APPLICATION**

(43) Date of publication:
**22.12.2021 Bulletin 2021/51**

(51) Int Cl.:
*A61B 5/00* (2006.01)       *G16H 50/20* (2018.01)

(21) Application number: **20305657.7**

(22) Date of filing: **16.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Institut Mines Telecom**
**91120 Palaiseau (FR)**

(72) Inventors:
• HOUMANI, Nesma
  **77000 Melun (FR)**
• ABAZID, Majd
  **75011 Paris (FR)**

(74) Representative: **Nony**
  **11 rue Saint-Georges**
  **75009 Paris (FR)**

(54) **METHOD FOR SELECTING FEATURES FROM ELECTROENCEPHALOGRAM SIGNALS**

(57)     A method for selecting features for the detection of a given brain condition, the method using a feature selector able to discriminate between at least two brain conditions, and using a plurality of electroencephalogram signals, relative to several brain electrodes and filtered on at least one frequency, the method comprising the following steps:

e) for each signal, computing at least one value quantifying brain activity for each brain electrode;

f) for each signal, performing a thresholding of said computed quantifying values according to at least one threshold percentage, thus forming at least one group of thresholded values corresponding to each signal;

g) using said feature selector to rank said thresholded values; and

h) based on this ranking, selecting at least one feature vector comprising at least one electrode, and one frequency and/or one threshold percentage, and corresponding to the best ranked thresholded values for the detection of said given brain condition.

EP 3 925 520 A1

## Description

### Technical field

The present invention relates to the technical field of signal processing relating to data analysis and neuroscience. In particular, the present invention deals with the analysis of electroencephalogram (EEG) signals based on a method to select features from EEG signals, and a method for discriminating among several types of brain conditions a subject suspected to have a brain condition using said features.

### Prior art

Electroencephalogram signals, relative to several brain electrodes, are mainly used to detect brain conditions, especially all forms of brain disorders such as Alzheimer's disease (AD), Mild Cognitive Impairment (MCI) or Subjective Cognitive Impairment (SCI). Among others, a reduction of the complexity of the EEG signals can be observed in case of subjects suffering from dementia, in particular from Alzheimer's disease, as well as a decrease of the synchrony measured in the EEG signals or the increase of functional disconnection between brain regions.

Conventionally, different EEG markers are extracted to quantify brain activity according to several aspects such as frequency bands, synchronization, coherence, time-frequency analysis, complexity measurement and functional graphs. Nevertheless, there is a difficulty to choose pertinent features in these signals because of the large amount of information they contain. In the literature, some studies analyse EEG signals either in the time domain or in specific standard frequency bands, as the delta, theta, alpha, beta, and gamma bands.

However, these known methods have some limitations. In particular, the extracted information from the EEG signals are smoothed, especially by averaging them over some or all of the brain electrodes. Then, the identification of the brain regions of interest is performed by specifying a group of brain electrodes depending on the position of each electrode on the head of the subject during the acquisition of the EEG signals. The choice of the electrodes belonging to a brain region or to another is study-dependent and thus the electrodes related to a brain region of interest may vary from one study to another.

Some recent methods are focusing on the analysis of brain functional connectivity based on graph theory to study the brain network topology. However, there is a need to define the connectivity threshold in order to only preserve relevant connections, considering the brain functions and/or the brain conditions under study.

Moreover, even if the study of EEG signals can be a convenient technique as it is non-invasive, cost-effective and potentially mobile, this method is not considered as a reliable imagery technique because of the complexity of the visual analysis, mainly because of the nature of the EEG signals that comprise a lot of small variations, have a low signal-to-noise ratio, and, often, an insufficient spatial resolution depending on the number of electrodes to identify brain functions.

### Objective of the invention

There is therefore a need to address, at least partially, the above-mentioned problems in order to use EEG signals for easily discriminating several brain conditions.

### Summary of the invention

The invention notably seeks to meet this objective and its subject, in one of its aspects, is a method for selecting features for the detection of a given brain condition, the method using a feature selector able to discriminate between at least two brain conditions, and using a plurality of electroencephalogram signals, relative to several brain electrodes and filtered on at least one frequency, the method comprising the following steps:

    a) for each signal, computing at least one value quantifying brain activity for each brain electrode;
    b) for each signal, performing a thresholding of said computed quantifying values according to at least one threshold percentage, thus forming at least one group of thresholded values corresponding to each signal;
    c) using said feature selector to rank said thresholded values; and
    d) based on this ranking, selecting at least one feature vector comprising at least one electrode, and one frequency and/or one threshold percentage, and corresponding to the best ranked thresholded values for the detection of said given brain condition.

The invention advantageously allows discriminating the following brain conditions: Alzheimer's disease (AD), mild cognitive impairment (MCI), subjective cognitive impairment (SCI) or healthy brain, but may also be used for other applications or other brain conditions. In particular, it could be used for cognitive training, cognitive impairment detection, neurofeedback therapy evaluation, or gaming control.

The invention is based on the quantification of the brain activity, in particular according to only one particular EEG marker, preferably the functional connectivity. Indeed, the filtering on preferably several frequencies and the thresholding of the values quantifying brain activity allow having a huge amount of information based on this single EEG marker. Thanks to the method according to the invention, relevant information, in term of electrode location, frequency and/or thresholding, can be selected from the huge amount of information extracted from EEG signals. The method according to the invention may also use the computation of several values quantifying brain activity corresponding to different EEG markers.

The possibility of extracting and exploiting a large amount of information which can result from a filtering at different frequencies and/or by using different thresholds, and for different electrodes, is particularly useful in the case of EEG signals acquired with few electrodes. It also allows selecting relevant information despite a low signal-to-noise ratio.

Furthermore, the method can be applied regardless the exploited EEG acquisition technology. In particular, Dry, Semi-dry, saline or gel-EEG systems may be exploited, for example CGX systems, Emotiv headsets, Deltamed digital EEG. This list is not exhaustive.

Preferably, the plurality of electroencephalogram signals is acquired for several subjects. For each subject, a plurality of electroencephalogram signals is acquired, each relative to a brain electrode. Preferably again, for at least a part of the subjects, better for all the subjects, the plurality of electroencephalogram signals is relative to the same brain electrodes. A subject is a person for which electroencephalogram signals are acquired regardless of whether this person is sick or not.

Preferably, electroencephalogram signals are acquired both for subjects with said given brain condition and subjects without said given brain condition.

The electroencephalogram signals may be relative to a range of 2 to 256 brain electrodes, especially to 30 brain electrodes.

The electroencephalogram signals are first acquired in the time domain and are then filtered on at least one frequency. Each signal may be filtered on at least two frequencies, better on at least four frequencies. Preferably, each signal is filtered on at least one frequency band, better on two frequency bands, better still on at least four frequency bands, even better on at least five frequency bands; the frequency bands being chosen amongst the well-known frequency bands delta, theta, alpha, beta and gamma.

Step a)

In step a), several values may be computed to quantify the brain activity for each signal, that is to say, for each electrode.

Preferably, in step a), one value is computed to quantify the functional connectivity between two brain electrodes arranged in a pair.

Said quantifying value is advantageously an epoch-based entropy computed between each pair of brain electrodes.

Said step a) is preferably carried out for each subject independently of each other, as quantifying the brain functional activity is subject dependent.

In particular, the invention allows quantifying the functional connectivity without specifying brain regions, as in the known methods.

In an embodiment, in particular for each subject and for each electrode, a combination of several intermediate values, for example computed by a mean, a variance, a standard deviation or a variance of the discrete cumulative function, is determined for the computation of the at least one quantifying value. For example, the intermediate values correspond to epoch-based entropies computed between each pair of brain electrodes, and the at least one brain quantifying value relative to a brain electrode is a combination of at least part of the epoch-based entropies relative to said brain electrode, allowing quantifying how the brain activity is distributed with regards to said brain electrode.

In a variant, said combination of several intermediate values is determined after the thresholding, the thresholded values thus being a combination of several intermediate values, in particular computed by a mean, a variance, a variance of the discrete cumulative function or a standard deviation.

In another embodiment, a vector of values can be associated to each electrode.

In a variant, said at least one computed quantifying value is a complexity marker, or a slowing marker.

The quantifying value is not limited to an epoch-based entropy. For example, it can also be a coherence measure, a phase synchrony measure, a granger causality measure, a Tsallis entropy, a sample entropy, a mutual information, or a graph parameter, for example, a clustering coefficient, a small word, a shortest path, an efficiency, a centrality, or a modularity. This list is not exhaustive.

Step b)

The invention may among other things bring to light which brain connections are relevant with regards to said given brain condition, in particular thanks to the thresholding performed at step b).

Preferably, the thresholding is performed for each subject independently from each other.

The thresholding is preferably a proportional thresholding, within a predefined range of percentage values and with a predefined step, the predefined range preferably being a range of percentage values between 0 % and 100 %, the predefined step preferably being less than or equal to 10 %, better being less than or equal to 1%.

The thresholded values may correspond to lower or higher values kept after said thresholding.

Alternatively, at step b), the at least one group of thresholded values can be a grouping of thresholded values from two thresholdings, for example a thresholding keeping the lower values, and a thresholding keeping the higher values.

Step c)

Each thresholded value is in particular related to a brain electrode, that is to say to a spatial location, to a frequency, and/or to a threshold percentage.

At step c), the thresholded values are ranked in order to determine at least one best feature vector for the discrimination of the given brain condition.

Such a ranking may highlight the thresholded values that distinguish a subject suffering from said given brain condition from a subject who does not suffer from said given brain condition.

Step d)

From the ranking determined in step c), at least one feature vector comprising at least one electrode, and one frequency and/or one threshold percentage is selected. Step d) may provide at least one feature vector comprising one electrode, one frequency band, and/or one threshold percentage.

The thresholded values relative to said at least one feature vector may make it possible to discriminate, on their own, subjects with a said given brain condition from the others.

In step d), a combination of several feature vectors may be selected, which allows reaching better classification performances.

In an exemplary embodiment, the method comprises a random probe method to determine the combination of vectors, especially with a predefined risk level at 10%, better at 5%. Such random probe method makes it possible to highlight the vectors that carry information, for the predefined risk level. The aim of this random probe method is to determine, for a predefined level of risk, which vector discriminates the brain conditions lower than a random variable, and thus can be assimilated to a noise signal.

Feature selector

In a preferred embodiment, the feature selector uses a Gram Schmidt algorithm, preferably using an Orthogonal Forward Regression (OFR), preferably again, in combination with a leave-one-out method, especially in case of sparse data. Said leave-one-out method may provide good results even if electroencephalogram signals from few subjects are acquired. Alternatively, a cross-validation method may be used. Several data set may be used defining in particular a train data set, a validation data set and a test data set.

In variants, the feature selector uses a correlation-based feature selection method, a logistic regression, a ranking by fisher ratio score, a decision tree, a principal component analysis, a linear discriminant analysis, a t-SNE (t-distributed stochastic neighbor embedding), a recursive feature elimination or a reverse sequential feature selection method. This list is not exhaustive.

Said feature selector may use at least pre-acquired values quantifying brain activity relative to several brain conditions in order to discriminate between at least two brain conditions.

Such pre-acquired values may be relative to the subject from which EEG signals are acquired, for example clinical data such as the presence of the given brain condition. The clinical data can also be relative to the gender, the age, or the presence of pathologies such as diabetes. Said pre-acquired values may also be relative to characteristics of encephalogram signals in relation to the given brain condition well known in the prior art, for example the sampling frequency, the duration of the acquisition, the technology of the acquisition system, the protocol of EEG acquisition (resting state with eyes closed, or with eyes opened, or under a visual and/or auditory stimuli).

In some embodiments, the feature selection comprises several sub-selections. Each sub-selection can select iteratively one feature of the at least one feature vector.

The selection of step d) may comprise:

- a first selection of at least one electrode corresponding to the best ranked thresholded values according to the ranking of step c);
- using a feature selector to rank the thresholded values corresponding to the selected electrode and to at least one frequency and/or one threshold percentage for the selection of at least a feature vector of one electrode, and one frequency and/or one threshold percentage corresponding to the best ranked thresholded values for the discrimination of said given brain condition.

In one embodiment, the feature selector used in the previous step is the feature selector used in step c) or a second feature selector. In particular, some selectors may be better for selecting some kind of features than others.

In one embodiment, the second selection may allow selecting at least one frequency or at least one thresholding percentage, and a third sub-selection may allow selecting respectively at least one thresholding percentage or at least one frequency.

Alternatively, the first sub-selection may allow selecting at least one frequency or at least one thresholding percentage, and the second and the third sub-selections may allow selecting at least one electrode, at least one frequency or at least one percentage thresholding, depending on the preceding sub-selections.

In another embodiment, several feature vectors may be selected iteratively. In this case, by means of iteratively processing sub-selections and removing the selected feature vector before processing new sub-selections, said selection may comprise the following steps:

D1) a first sub-selection selecting one electrode;
D2) a second sub-selection selecting one frequency and one percentage thresholding;

D3) removing the selected feature vector comprising the electrode, the frequency and the percentage thresholding from the ranking; and go back to step D1).

Preferably, a condition is defined to stop the iterations, such as a predefined number of selected feature vectors and/or Gini index(es) and/or the use of probe random method and/or performance assessment.

Discriminating method

The invention also relates to a method for discriminating among several types of brain conditions a subject suspected to have a brain condition, at least based on electroencephalogram signals of the subject relative to several brain electrodes and filtered on at least one frequency, the method using at least a classifier, especially a linear SVM classifier, trained beforehand to learn to discriminate said brain condition based at least on a plurality of feature vectors previously-selected by using the feature selection method according to the invention, the method comprising the following steps:

    a) for each signal, computing at least one value quantifying brain activity for each brain electrode;
    b) performing a thresholding of said computed quantifying value(s) according to at least a threshold percentage selected by the feature selection method according to the invention;
    c) forming a subject feature vector comprising at least one electrode, and one frequency and/or one threshold percentage based on the feature selection method according to the invention, and
    d) operating the trained classifier on said subject feature vector to discriminate said subject among several types of brain conditions.

Such classification method allows determining, quickly and efficiently, if the subject has a given brain condition, especially from signals acquired by using few brain electrodes.
Said classifier may be supervised or unsupervised. Said classifier may use support vector machine(s), neural networks, linear discriminant analysis, decision trees, random forest, hidden Markov models, gaussian mixture models, regression, k-means, hierarchical clustering, or DBSCAN (density-based spatial clustering of applications with noise). This list is not exhaustive.
Preferably, the method for discriminating among several types of brain conditions a subject suspected to have a brain condition comprises the transmission, to the subject or a medical expert, of an output of the trained classifier, for example a score estimating whether said subject has said brain condition, or several scores, each corresponding to one brain condition.
Said score(s) may be in the form of a probability or a percentage. Said score(s) may be in the form of a numerical value, for example a value comprised between 0 and 10. In a variant, said score(s) are in the form of a letter, especially showing that a subject is thought to belong to a group of people having a given brain condition, for example group A, group B, group C, and so on.
In yet another variant, said score may be a class, such as "AD", "SCI", or "MCI", or a combination thereof, for example a probability that the subject belongs to the "AD" class, and/or a probability that the subject belongs to the "SCI" class, and/or a probability that the subject belongs to the "MCI" class.
Said score(s) may be transmitted to a user by any suitable mean, for example by being displayed on a screen of an electronic device, printed, or by vocal synthesis.
Said score(s) may be used as entry value in another program, and/or may be combined to other information, for example clinical and/or biological data.
Scores obtained for a same subject at different times may be compared in order to assess the evolution of their brain condition. Successive classification scores may be stored on the memory of an electronic device.

Computer program product

Such methods according to the invention are advantageously performed by means of computer programs, automatically on any electronic device comprising a processor, especially a computer.
The invention also relates to a computer program product for selecting features for the detection of a given brain condition, by using a feature selector able to discriminate between at least two brain conditions, and a plurality of electroencephalogram signals, relative to several brain electrodes and filtered on at least one frequency, the computer program product comprising a support and stored on this support instructions that can be read by a processor, these instructions being configured, when executed, for:

    a) for each signal, computing at least one value quantifying brain activity for each brain electrode;
    b) for each signal, performing a thresholding of said computed quantifying values according to at least one threshold percentages, thus forming at least one group of thresholded values corresponding to each signal;
    c) using said feature selector to rank said thresholded values; and
    d) based on this ranking, selecting at least one feature vector comprising at least one electrode, and one frequency and/or one threshold percentage, and corresponding to the best ranked thresholded values for the detection of said given brain condition.

The invention also relates to a computer program product for discriminating among several types of brain conditions a subject suspected to have a brain condition, at least based on electroencephalogram signals of the subject relative to several brain electrodes and filtered on at least one frequency, the method using at least a classifier, especially a linear SVM classifier, trained beforehand to learn to discriminate said brain condition based at least on a plurality of feature vectors previously-selected by using the feature selection method according to the invention, the computer program product comprising a support and stored on this support instructions that can be read by a processor, these instructions being configured, when executed, for:

a) for each signal, computing at least one value quantifying brain activity for each brain electrode;

b) performing a thresholding of said computed quantifying value(s) according to at least a threshold percentage selected by the feature selection method according to the invention;

c) forming a subject feature vector comprising at least one electrode, and one frequency and/or one threshold percentage based on the feature selection method according to the invention, and

d) operating the trained classifier on said subject feature vector to discriminate said subject among several types of brain conditions.

Such computer program products may be performed on a mobile phone, a tablet, a computer, mobile or fixe, or any electronic device comprising a processor.

## Brief description of the figures

Other features and advantages of the invention will become more apparent on reading the following detailed description and on studying the attached drawing in which:

[Fig 1] figure 1 describes different steps of an example of method according to the invention;

[Fig 2] figure 2 describes different steps of another example of method according to the invention;

[Fig 3] figure 3 describes an example of spatial location of brain electrodes during the acquisition of electroencephalogram signals;

[Fig 4] figure 4 illustrates several groups of thresholded values;

[Fig 5] figure 5 is an example of a classification performance of a linear SVM classifier used to discriminate subjective cognitive impairment subjects and Alzheimer's disease subjects, according to the invention;

[Fig 6] figure 6 illustrates part of the modelling of an encephalogram signal to compute epoch-based entropy;

[Fig 7] figure 7 is an example of the implementation of a method according to the invention; and

[Fig 8] figure 8 describes different exemplary steps of a method according to the invention.

## Detailed description

### Acquisition of electroencephalogram signals

The method is based on an analysis of several electroencephalogram signals, each signal being acquired from a brain electrode positioned on the head of a subject. Usually, several EEG signals are acquired simultaneously from N brain electrodes positioned on the head of a subject.

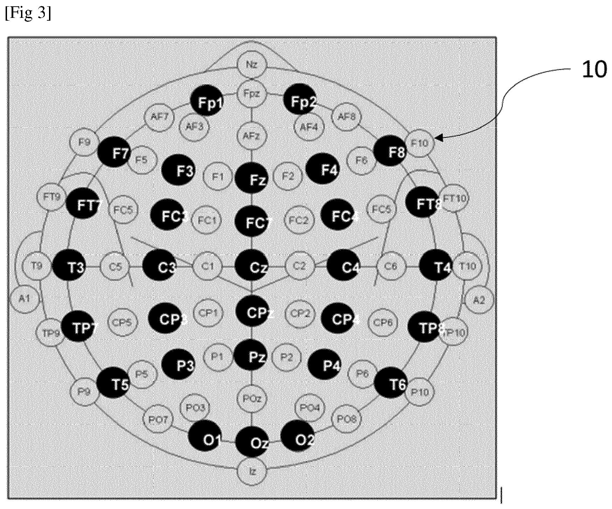

Figure 3 illustrates an example of an acquisition of EEG signals with 30 brain electrodes 10. More particularly, figure 3 represents a top view of the head of a subject with 30 electrodes 10 positioned according to the international 10-20 system. Conventionally, the EEG signals are acquired when the subject is resting with his eyes closed.

Such acquisition may be performed for several subjects M, preferably some of said subjects suffering from a given brain condition and others who do not suffer from said given brain condition.

Each EEG signal is then filtered on at least one frequency or frequency band, preferably on at least four frequencies bands, for example on the well-known frequency bands delta, theta, alpha and beta.

The lower limit and higher limit of these frequency bands can vary but they generally are respectively around [0.1 Hz-4 Hz], [4 Hz-8 Hz], [8 Hz-12 Hz] and [12 Hz-30 Hz]. Each EEG signal can also be filtered on the gamma frequency band, which generally corresponds to frequencies greater than 30 Hz.

### Quantifying brain activity

At step a), for each subject and for each frequency band, the functional connectivity between two brain electrodes may be computed between each pair of brain electrodes, preferably *via* the computation of epoch-based entropies.

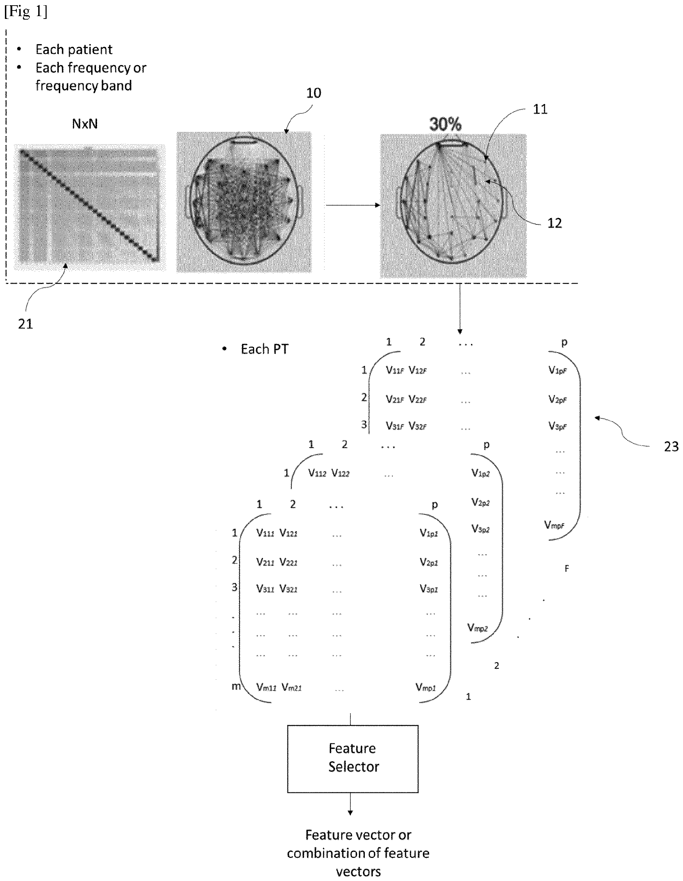

As illustrated in figure 1, for each subject and for each frequency band, a matrix 21 comprising N*N epoch-based entropies may be computed, N being the number of brain electrodes used during the acquisition of the EEG signals for a subject.

Based on the epoch-based entropies, graph theory methods may be applied. In particular, graph theory provides a spatial visualisation that can highlight the relationship between the structure and the process of the brain. In figure 1, the representation 10 of the epoch-based entropies allows visualizing the connections 12 between each brain electrodes 11.

Preferably, before computing the representation 10, the quantifying values, in this example the epoch-based entropies, are normalized, for example following a min-max method considering at least part of the quantifying values of all the subjects and all frequencies or frequency bands, better considering all of the quantifying values of all subjects and all frequencies or frequency bands. The quantifying values may be comprised between 0 and 1.

The N epoch-based entropies relative to one electrode may then be combined, for example by measuring a variance of the discrete cumulative function, in order to have one quantifying brain activity value per electrode, per frequency band, and per subject. For each frequency band and for each subject, the quantifying values may thus be illustrated by a vector 1*N.

At the end of step a), a set of quantifying values is obtained comprising at least one quantifying value for each electrode, preferably for each frequency band and for each subject.

Epoch-based entropy measure

An advantage of the epoch-based entropy is that it allows estimating the complexity of EEG signals locally over time but also spatially by estimating the inter-channel complexity.

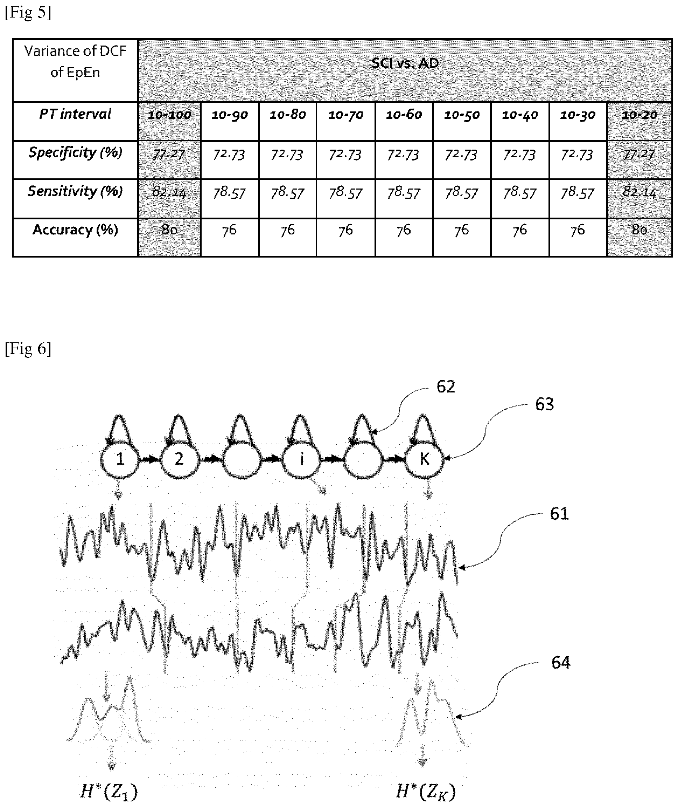

To compute the epoch-based entropy, EEG signals are modelled by a continuous left-to-right Hidden Markov Model (HMM). Such modelling of two EEG signals is illustrated in figure 6. The HMM comprises states 63 and transitions 62, the states 63 corresponding to stationary parts of an EEG signal and the transitions 62 corresponding to the variations of the EEG signal. An EEG signal recorded from a subject is considered as a succession of epochs obtained by segmenting the signal 61, for example using a Viterbi algorithm by means of the corresponding subject HMM. Thus, each obtained epoch corresponds to a state of the HMM and contains a given number of observations (sample points). For each epoch $S_i$, the probability density function is modelled by a mixture of $M_G$ Gaussian functions 64.

Then each observation z in a given epoch $S_i$ is considered as a realization $Z_i$ of a random variable Z that follows a given observation probability distribution $P_i(z)$ modelled by the mixture of Gaussians 64. Consequently, each stationary epoch of the signal is associated to a random variable, and the entropy $H^*(Z_i)$ of the epoch $\mathbf{S_i}$ is that of an ensemble of realizations of $Z_i$:

$$H^*(Z_i) = -\sum_{z \in S_i} P_i(z) . log_2(P_i(z))$$

By averaging the entropy over the K epochs of the EEG signal 61 of a subject, an entropy-based complexity value EpEn(Z) of the signal 61, called "epoch-based entropy", is obtained as:

$$EpEn(Z) = \frac{1}{K} \sum_{i=1}^{K} H^*(Z_i)$$

To model the inter-relations between EEG time series, filtered in a frequency or different frequencies or different frequency bands, recorded from N brain electrodes, an HMM is trained for each subject on a set of N EEG signals recorded from N brain electrodes. At a time t, a hidden state emits a *N*-dimensional observation vector. By applying the Viterbi algorithm, each EEG signal is segmented into K epochs, and the entropy $H^*(Z_i)$ of each epoch $S_i$ is computed considering the probability density estimated by the HMM on the observations of the *K* epochs. Although all *K* epochs are matched between EEG channels, the model does not constrain these epochs to be of equal length for all channels. Finally, by averaging the entropy over all the *K* epochs, an epoch-based entropy value associated to the multi-channel EEG of the subject is computed.

The quantifying value is not limited to an epoch-based entropy. For example, it may be a graph parameter such as a clustering coefficient used as a segregation measure and defined as follows: $C_i = \frac{2t_i}{K_i(K_i - 1)}$ *avec* $t_i = \frac{1}{2} \sum_{j,k} a_{i,j} a_{j,k} a_{i,j}$ *et* $K_i = \sum_{j=1}^{n} a_{ij}$, $t_i$ being the number of triangles around a vertex *i* and $K_i$ being the degree of a vertex *i* and the sum of afferent and efferent connections.

Thresholding

At step b), starting from all quantifying values for each electrode of a subject, a thresholding step is performed, for each frequency band separately.

Preferably, such thresholding is a percentage thresholding (PT), as represented in figure 1 with a PT of 30%.

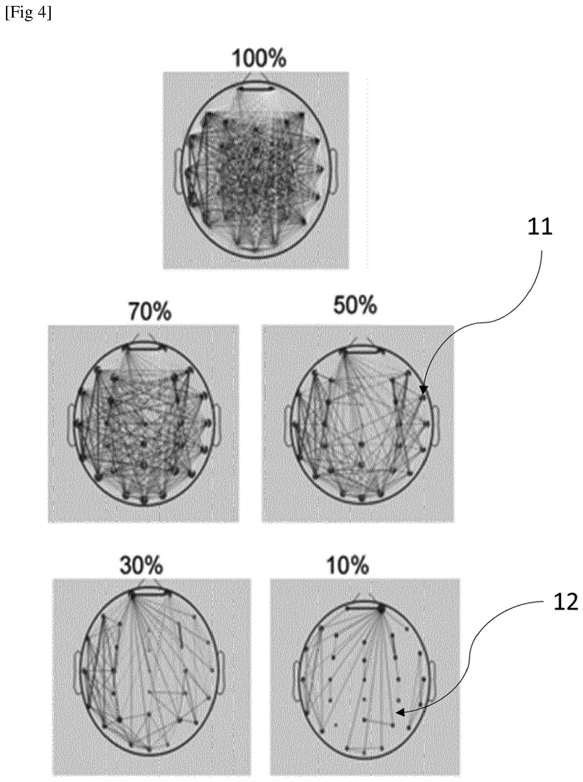

Other examples of percentage thresholdings are illustrated in figure 4, within a predefined range of percentage values and with a predefined step, the predefined range preferably being a range of percentage values between 10 % and 100 %, the predefined step being 30 %.

For each subject, for each frequency band, 5 groups of thresholded values are thus obtained in the example of figure 4. Each line 12 represents a connection between two electrodes 11 and the intensity colour corresponds to the quantifying values, for example the epoch-based entropy computed between said two electrodes.

In the example corresponding to the 100% thresholding, 100% of the quantifying values are kept, forming a group of thresholded values corresponding to a PT equal to 100%. In the example corresponding to the 70% thresholding, the 70% highest quantifying values are kept, forming another group of thresholded values corresponding to a PT equal to 70%. In another embodiment, the 70% lowest quantifying values can be kept. In the example corresponding to a PT equal to 50%, the 50% highest quantifying values are kept. In another embodiment, the 50% lowest quantifying values can be kept. In the example corresponding to a PT equal to 30%, the 30% highest quantifying values are kept. In another embodiment, the 30% lowest quantifying values can be kept. In the last example corresponding to a PT equal to 10%, the 10% highest quantifying values are kept. In another embodiment, the 10% lowest quantifying values can be kept.

Feature selector

At step c), in the example of figure 1, the thresholded values 23 relative to all frequency bands F, all PT, and all subjects M, are then ranked using a feature selector, preferably using a Gram Schmidt algorithm, and an Orthogonal Forward Regression.

In particular, the feature selector comprises the following steps:

C1) selection of a feature vector $f_i$ that best discriminate the brain conditions;
C2) projection of the output vector onto the null space of the selected feature vector and orthogonalization of the rest of the feature vectors using Gram Schmidt algorithm;
C3) removing the selected feature vector $f_i$ from the list of the feature vectors;
C4) returning to C1 until a condition is met.

Said feature selector allows highlighting which thresholded values can discriminate the given brain condition from other brain conditions by ranking said thresholded values.

Preferably a leave-one-out procedure is used in case of sparse data, that is to say when EEG signals of few subjects are acquired to apply the method as described.

In particular, the feature selector uses at least pre-acquired values quantifying brain activity relative to several brain conditions in order to discriminate between at least two brain conditions. For example, pre-acquired values may correspond to the knowledge of which subjects have the given brain condition and which subjects have not the given brain condition. Pre-acquired values may also be clinical, medical or personal data relative to the subject, such as age, gender, educational level, profession, family situation, aetiology, subject history.

At step d), from the ranking of said thresholding values, a feature vector or better a combination of feature vectors is selected. For example, the following triplet can be selected (alpha, 10, FC4) that correspond to the thresholded values obtained for a EEG signal acquired with an electrode positioned on the FC4 location, according to the International 10-20 system, filtered on the frequency band alpha and thresholded with a PT of 10%. In another embodiment, a combination of vectors may be selected such as the following example [(alpha, 10, FC4); (delta, 20, T6); (beta, 20, FC3); (beta, 30, FP2); (theta, 100, T3)].

Preferably, the number of feature vectors selected is defined by using a random probe method, preferably again with a predefined risk level of maximum 10%, better of maximum 5%. For this purpose, random realizations of thresholding are generated, for example at least 100 random realizations, or better at least 1000 random realizations of thresholded values and concatenate with real thresholded values. The random thresholded values and the real thresholded values are then ranked as in step c). Feature vectors are then selected according to the predefined risk level. The estimation of the risk may be the condition to be met at step C4. The feature vector may be kept although it might be less relevant than the probe.

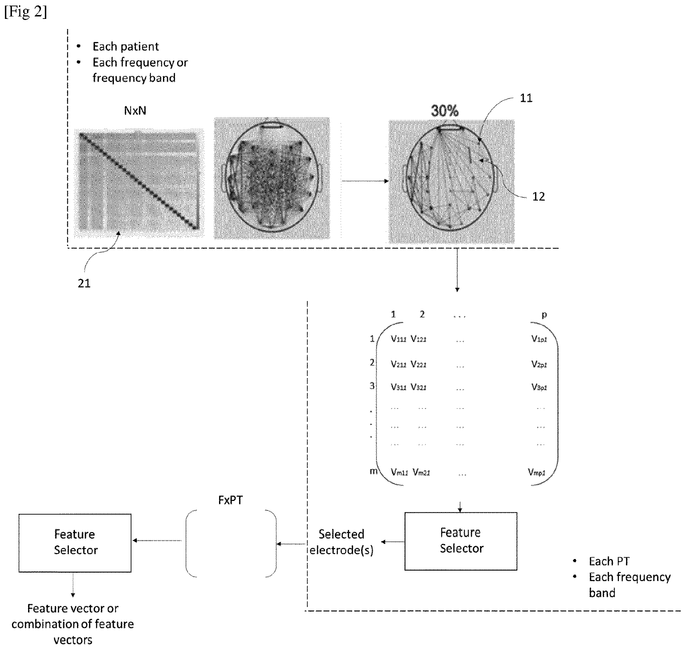

Figure 2 illustrates another preferred embodiment of the method similar to the one described in relation with figure 1, in which the selection at step d) comprises two successive selections:

- a first selection of at least one brain electrode corresponding to a best ranked thresholded values according to the ranking of step c), being performed for each frequency band and each PT;
- using a feature selector to rank the thresholded values corresponding to the selected brain electrode and to at least one frequency and/or one threshold percentage, and
- selecting at least a feature vector of one brain electrode, and one frequency and/or one threshold percentage corresponding to the best ranked thresholded values for the detection of said given brain condition.

In one embodiment, the feature selector is the feature selector used in step c) or a second feature selector. In a preferred embodiment, both feature selectors are Orthogonal Forward Regression algorithm using Gram Schmidt algorithms and used with a leave-one-out procedure. Preferably again, the selection of the number of feature vectors is defined using a random probe method.

In one embodiment, steps b) and c) may be repeated to compare different ranges of PT, as the feature selection is sensitive to the considered ranges of PT, for example considering the range 0% to 100%, 0% to 90%, 0% to 80% and so on until 0% to 20%, with a step of 10% on the all range 0% to 100%. One can also consider only part of the range, for example 10% to 100% with a step of 20%: 10% to 100%, 10% to 80%, 10% to 60%, 10% to 40%, and 10% to 20%.

The number of thresholded values depends on the range of the PT and on the predefined step. For example, when considering a 10%-60% range with a predefined step of 10%, for the four frequency bands alpha, beta, theta and delta, 24 cases are considered for each electrode for each subject.

Different feature vectors or combination of feature vectors may be selected depending on the range and the step of the PT.

Example

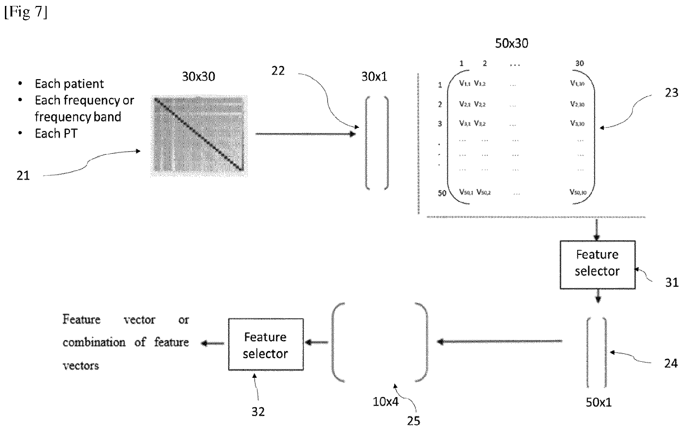

A preferred embodiment of the method for selecting features for the discrimination of a given brain condition is illustrated in figure 7.

In this example, EEG signals of 50 subjects are acquired, said EEG signals being relative to 30 brain electrodes. Among those 50 subjects, 28 subjects are AD subjects and 22 subjects are SCI subjects.

Each EEG signal is filtered on the following frequency bands: delta (0.1-4Hz), theta (4-8Hz), alpha (8-12Hz) and beta (12-30Hz).

For each subject and for each frequency band, epoch-based entropy is computed between each pair of brain electrodes, forming a 30x30 matrix comprising the computed quantifying values.

Each value of each matrix is then thresholded, by applying a percentage thresholding, within a range 10%-100% with a step of 10%. For each subject and for each frequency band, 10 groups of thresholded values are obtained, each comprising 30x30 thresholded values. The quantifying values that have not been retained after the thresholding step are then replaced by a neutral value, e.g. a mean value or a zero value for the set of values considered.

Each matrix 21 comprising the thresholded values are then converted to a vector 22, of size 30x1, wherein, in this example, each value relative to a brain electrode is the variance of the discrete cumulative function (variance of DCF) of the thresholded values relative to said electrode. The vectors 22 relative to each of the 50 subjects are then grouped into a matrix 23 of size 50x30.

For each frequency band and for each PT, the thresholded values are ranked using a feature selector 31, a Gram Schmidt algorithm following a leave-one-out procedure in this example. This ranking allows selecting at least one brain electrode, that is to say a vector 24 of size 50x1, said at least one brain electrode being relevant for discriminating the two brain conditions, namely AD and SCI, for the given frequency band and the given PT. In this example, this step is therefore repeated 40 times, for each of the 4 frequency bands and each of the 10 groups of thresholded values, forming a matrix 25 of size 50x40 that regroups the 40 selected vectors 24.

Another ranking is performed based on the selected thresholded values of matrix 25 comprising the thresholded values relative to the selected brain electrodes, using a feature selector 32 being a Gram Schmidt algorithm following a leave-one-out procedure. This ranking allows selecting at least one frequency band and at least one PT, thus forming at least one feature vector composed of a brain electrode, or a location, a frequency band and a percentage threshold step, for example the brain electrode positioned on the location FC4, according to the international 10-20 system, the frequency band alpha and the thresholding 10%.

In a preferred embodiment, to select the at least one frequency band and the at least one PT, a random probe method is applied, defining the number of feature vectors to keep. Say differently, the random probe method allows determining which triple, "brain electrode - frequency band - percentage threshold", discriminates both brain conditions more than a random variable, considering a predefined acceptable risk level. To do that, several random variables are generated and ranked together with the thresholded values of matrix 25.

The selection of the at least one feature vector is preferably performed successively and comprises the following steps:

- pre-selection of a feature vector;
- determining whether this pre-selection respects the predefined acceptable risk level;
- if the pre-selection respects the predefined acceptable risk level:

  ◦ selection of the feature vector,
  ◦ feature vector removable from matrix 25,
  ◦ repetition at the pre-selection.

- if the pre-selection does not respect the predefined acceptable risk level, stopping the feature vector selection.

The number of selected feature vectors may vary but all selected feature vectors are advantageously significant.

Performances of the feature selection method

The table of figure 5 comprises performances of classifications carried out from feature vectors selected from a method as previously described for different ranges of PT considered. For each different ranges of PT considered, several percentage thresholdings are performed, within each range from 10% to 100% with a step of 10%. In this example, the classifications are performed with a linear support vector machine (SVM), in order to discriminate SCI subjects from AD subjects.

The table shows that an accuracy of 80% is obtained considering all the 10 PT ranges by selecting the following combination of feature vectors [(alpha, 10, FC4); (delta, 20, T6); (beta, 20, FC3); (beta, 30, FP2); (theta, 100, T3)] or considering only 2 PT ranges (10% to 20%, with a step of 10%) by selecting the following combination of feature vectors [(alpha, 10, FC4); (delta, 20, T6); (beta, 20, FC3); (theta, 10, TP7)].

The method allows reaching a good classification performance, comparable to the known methods, by combining different PT, that is to say different network densities scales, different frequency bands and different electrodes, that is to say different locations on the brain, furthermore by using only one EEG marker, in this example the functional connectivity via the computation of the epoch-based entropy.

Discriminating method

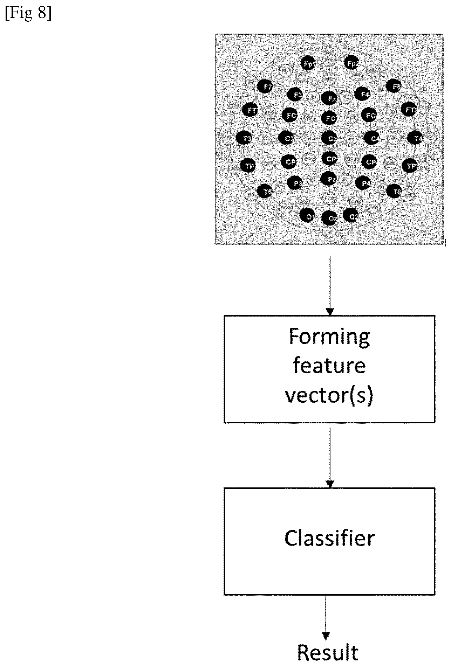

Figure 8 illustrates steps of a method for discriminating, among several types of brain conditions, a subject suspected to have a brain condition according to the invention.

Such a method uses a classifier, for example a linear Support Vector Machine (SVM), which may determine a score estimating whether said subject has said brain condition or not.

For this purpose, the classifier is trained beforehand to learn to discriminate said brain condition based at least on a plurality of feature vectors previously selected by using the method for selecting features for the discrimination of a given brain condition according to the invention.

Once the classifier is trained, it is possible to estimate, for subjects suspected to have said brain condition, if they have or not said brain condition, from feature vectors issued from the analysis of acquired EEG signals.

As such, for a subject, EEG signals of said subject are acquired and filtered on at least one frequency or frequency band, as previously explained. The acquisition and the filtering may depend on which feature vector(s) are previously selected for the training of the classifier.

At step a), for each EEG signal, at least one value quantifying brain activity for each brain electrode is computed, for example an epoch-based entropy is computed between each pair of brain electrodes. The computation of the quantifying brain activity may depend on which feature vector(s) are previously selected for the training of the classifier.

At step b), a percentage threshold is then performed on the computed quantifying values depending on which feature vector(s) are previously selected for the training of the classifier.

Step c) comprises the formation, from the thresholded values, of at least one feature vector which is given, at step d), as an entry to the classifier, said feature vector comprising at least one brain electrode, and one frequency or frequency band and/or one threshold percentage.

Preferably, the output of the classifier is a score estimating whether said subject has said brain condition, or several scores, each corresponding to one brain condition. For example, a probability or a percentage which may be transmitted to the subject and/or to a medical expert. It may also be a class, such as "AD", "SCI", or "MCI", or a combination thereof, for example a probability that the subject belongs to the "AD" class, and/or a probability that the subject belongs to the "SCI" class, and/or a probability that the subject belongs to the "MCI" class.

The transmission of such result may be made electronically, for example send by email or displayed on a screen, for example on a computer, a mobile phone or a tablet. Alternatively, or additionally, it can be printed.

The invention is not limited to the examples described above.

In particular, the method is not limited to a discrimination between AD subjects and SCI subjects.

The acquisition of the EEG signals is not limited to an acquisition realized with 30 brain electrodes.

The feature selector is not limited to a Gram Schmidt algorithm in combination to a leave-one-out procedure. The Gram Schmidt algorithm may be in combination with a k-folds procedure. Alternatively, it may be a correlation-based feature selection method, a logistic regression, a ranking by fisher ratio score, a decision tree, a principal component analysis, a linear discriminant analysis, a t-SNE (t-distributed stochastic neighbor embedding), a recursive feature elimination or a reverse sequential feature selection method, in combination with a leave-one-out procedure or a k-folds procedure.

In particular, the lower limit and higher limit of the frequency bands are not limited to those described. Indeed, the lower limit and the higher limit of these well-known frequency bands can advantageously be defined as follows:

- the lower limit of the frequency band delta can be comprised between 0.05 Hz and 1 Hz and the higher limit of the frequency band delta can be comprised between 2 Hz and 5 Hz;
- the lower limit of the frequency band theta can be comprised between 3 Hz and 5 Hz and the higher limit of the frequency band theta can be comprised between 6 Hz and 9 Hz;
- the lower limit of the frequency band alpha can be comprised between 6 Hz and 9 Hz and the higher limit of the frequency band alpha can be comprised between 10 Hz and 17 Hz;
- the lower limit of the frequency band beta can be comprised between 10 Hz and 17 Hz and the higher limit of the frequency band beta can be comprised between 25 Hz and 35 Hz;
- the lower limit of the frequency band gamma can be comprised between 25 Hz and 35 Hz and the higher limit of the frequency band gamma can reach 100 Hz

**Claims**

1. A method for selecting features for the detection of a given brain condition, the method using a feature selector able to discriminate between at least two brain conditions, and using a plurality of electroencephalogram signals, relative to several brain electrodes and filtered on at least one frequency, the method comprising the following steps:

a) for each signal, computing at least one value quantifying brain activity for each brain electrode;

b) for each signal, performing a thresholding of said computed quantifying values according to at least one threshold percentage, thus forming at least one group of thresholded values corresponding to each signal;

c) using said feature selector to rank said thresholded values; and

d) based on this ranking, selecting at least one feature vector comprising at least one electrode, and one frequency and/or one threshold percentage, and corresponding to the best ranked thresholded values for the detection of said given brain condition.

2. The method of claim 1, wherein, in step a), said at least one computed value quantifies the functional connectivity between two brain electrodes arranged in a pair.

3. The method of any one of claim 1 or 2, wherein, in step a), said at least one quantifying value is an epoch-based entropy computed between each pair of brain electrodes.

4. The method of any one of the preceding claims, wherein the at least one quantifying value is a combination of several intermediate values, in particular computed by a mean, a variance, a variance of the discrete cumulative function or a standard deviation.

5. The method of any one of the preceding claims, wherein said feature selector uses at least pre-acquired values quantifying brain activity relative to several brain conditions in order to discriminate between at least two brain conditions.

6. The method of any one of the preceding claims, wherein said brain conditions are Alzheimer's disease (AD), mild cognitive impairment (MCI), subjective cognitive impairment (SCI) or healthy brain.

7. The method of any one of the preceding claims, wherein the selection of step d) comprises:

- a first selection of at least one electrode corresponding to the best ranked thresholded values according to the ranking of step c);
- using a feature selector to rank the thresholded values corresponding to the selected electrode and to at least one frequency and/or one threshold percentage, for the selection of at least a feature vector of one electrode, and one frequency and/or one threshold percentage corresponding to the best ranked thresholded values for the discrimination of said given brain condition.

8. The method of the preceding claim, wherein the feature selector is the feature selector used in step c) or a second feature selector.

9. The method of any one of the preceding claims, wherein, at step b), the thresholding is a proportional thresholding, within a predefined range of percentage values and with a predefined step, the predefined range preferably being a range of percentage values between 0 % and 100 %, the predefined step preferably being less than or equal to 10 %.

10. The method of any one of the preceding claims, wherein the electroencephalogram signals are filtered on at least one frequency band, better on two frequency bands, better still on at least four frequency bands, even better on at least five frequency bands; said frequency band being chosen amongst the bands delta, theta, alpha, beta, and gamma.

11. The method of any one of the preceding claims, wherein step d) provides at least one feature vector comprising one electrode, one frequency band, and one threshold percentage.

12. The method of any one of the preceding claims, wherein the feature selector uses a Gram Schmidt algorithm, preferably using an Orthogonal Forward Regression (OFR), preferably in combination with a leave-one-out method.

13. The method of any one of claims 1 to 11, wherein the feature selector uses a correlation-based feature selection method, a logistic regression, a ranking by fisher ratio score, or a reverse sequential feature selection method.

14. The method of any one of the preceding claims except claim 2, wherein said at least one computed value is a complexity marker, or a slowing marker.

15. The method of any one of the preceding claims except claim 3, wherein, in step a), said at least one quantifying value is a coherence measure, a phase synchrony measure, a granger causality measure, a Tsallis entropy, a sample entropy, or a mutual information, or a graph parameter, for example, a clustering coefficient, a small word, a shortest path, an efficiency, a centrality, or a modularity.

16. The method of any one of the preceding claims, wherein, in step d), a combination of several feature vectors is selected.

17. The method of any one of the preceding claims, wherein the electroencephalogram signals are relative to a range of 2 to 256 brain electrodes, especially to 30 brain electrodes.

18. A method for discriminating among several types of brain conditions a subject suspected to have a brain condition, at least based on electroencephalogram signals of the subject relative to several brain electrodes and filtered on at least one frequency, the method using at least a classifier, especially a linear SVM classifier, trained beforehand to learn to discriminate said brain condition based at least on a plurality of feature vectors previously-selected by using the feature selection method of claims 1 to 17, the method comprising the following steps:

> a) for each signal, computing at least one value quantifying brain activity for each brain electrode;
> b) performing a thresholding of said computed quantifying value(s) according to at least a threshold percentage selected by the feature selection method of claims 1 to 17;
> c) forming a subject feature vector comprising at least one electrode, and one frequency and/or one threshold percentage based on the feature selection method of claims 1 to 17, and
> d) operating the trained classifier on said subject feature vector to discriminate said subject among several types of brain conditions.

19. Computer program product for selecting features for the detection of a given brain condition, by using a feature selector able to discriminate between at least two brain conditions, and a plurality of electroencephalogram signals, relative to several brain electrodes and filtered on at least one frequency, the computer program product comprising a support and stored on this support instructions that can be read by a processor, these instructions being configured, when executed, for:

> a) for each signal, computing at least one value quantifying brain activity for each brain electrode;
> b) for each signal, performing a thresholding of said computed quantifying values according to at least one threshold percentages, thus forming at least one group of thresholded values corresponding to each signal;
> c) using said feature selector to rank said thresholded values; and
> d) based on this ranking, selecting at least one feature vector comprising at least one electrode, and one frequency and/or one threshold percentage, and corresponding to the best ranked thresholded values for the detection of said given brain condition.

[Fig 1]

- Each patient
- Each frequency or frequency band

NxN

10

30%

11

12

21

- Each PT

|   | 1 | 2 | ... | p |
|---|---|---|-----|---|
| 1 | $V_{11F}$ $V_{12F}$ | | ... | $V_{1pF}$ |
| 2 | $V_{21F}$ $V_{22F}$ | | ... | $V_{2pF}$ |
| 3 | $V_{31F}$ $V_{32F}$ | | ... | $V_{3pF}$ |
| | | | ... | ... |
| | | | | ... |
| | | | | $V_{mpF}$ |

F

23

|   | 1 | 2 | ... | p |
|---|---|---|-----|---|
| 1 | $V_{112}$ $V_{122}$ | | ... | $V_{1p2}$ |
| | | | | $V_{2p2}$ |
| | | | | $V_{3p2}$ |
| | | | | ... |
| | | | | ... |
| | | | | $V_{mp2}$ |

2

|   | 1 | 2 | ... | p |
|---|---|---|-----|---|
| 1 | $V_{111}$ $V_{121}$ | | ... | $V_{1p1}$ |
| 2 | $V_{211}$ $V_{221}$ | | ... | $V_{2p1}$ |
| 3 | $V_{311}$ $V_{321}$ | | ... | $V_{3p1}$ |
| . | ... | ... | ... | ... |
| . | ... | ... | ... | ... |
| . | ... | ... | ... | ... |
| m | $V_{m11}$ $V_{m21}$ | | ... | $V_{mp1}$ |

1

Feature Selector

Feature vector or combination of feature vectors

[Fig 2]

[Fig 3]

[Fig 4]

[Fig 5]

| Variance of DCF of EpEn | SCI vs. AD | | | | | | | | |
|---|---|---|---|---|---|---|---|---|---|
| PT interval | 10-100 | 10-90 | 10-80 | 10-70 | 10-60 | 10-50 | 10-40 | 10-30 | 10-20 |
| Specificity (%) | 77.27 | 72.73 | 72.73 | 72.73 | 72.73 | 72.73 | 72.73 | 72.73 | 77.27 |
| Sensitivity (%) | 82.14 | 78.57 | 78.57 | 78.57 | 78.57 | 78.57 | 78.57 | 78.57 | 82.14 |
| Accuracy (%) | 80 | 76 | 76 | 76 | 76 | 76 | 76 | 76 | 80 |

[Fig 6]

$$H^*(Z_1) \qquad\qquad H^*(Z_K)$$

[Fig 7]

[Fig 8]

Forming
feature
vector(s)

Classifier

Result

Europäisches
Patentamt

European
Patent Office

Office européen
des brevets

# EUROPEAN SEARCH REPORT

Application Number

EP 20 30 5657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/296569 A1 (SHAHAF GODED [IL] ET AL) 22 November 2012 (2012-11-22) * paragraphs [0011], [0021], [0027], [0039], [0138], [0288] - [0309] * | 1-19 | INV. A61B5/00 G16H50/20 |
| A | DVORAK DINO ET AL: "Cognitive Behavior Classification From Scalp EEG Signals", IEEE TRANSACTIONS ON NEURAL SYSTEMS AND REHABILITATION ENGINEERING, IEEE SERVICE CENTER, NEW YORK, NY, US, vol. 26, no. 4, 1 April 2018 (2018-04-01), pages 729-739, XP011680555, ISSN: 1534-4320, DOI: 10.1109/TNSRE.2018.2797547 [retrieved on 2018-04-05] * the whole document * | 1-19 | |
| A | SONG ZHENXI ET AL: "Biomarkers for Alzheimer's Disease Defined by a Novel Brain Functional Network Measure", IEEE TRANSACTIONS ON BIOMEDICAL ENGINEERING, IEEE SERVICE CENTER, PISCATAWAY, NJ, USA, vol. 66, no. 1, 1 January 2019 (2019-01-01), pages 41-49, XP011700889, ISSN: 0018-9294, DOI: 10.1109/TBME.2018.2834546 [retrieved on 2018-12-18] * the whole document * -/-- | 1-19 | TECHNICAL FIELDS SEARCHED (IPC) A61B G16H |

The present search report has been drawn up for all claims

| Place of search | Date of completion of the search | Examiner |
|---|---|---|
| Berlin | 30 November 2020 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 30 5657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHOEB A ET AL: "Patient-specific seizure onset detection", EPILEPSY AND BEHAVIOR, ACADEMIC PRESS, SAN DIEGO, CA, US, vol. 5, no. 4, 1 August 2004 (2004-08-01), pages 483-498, XP004775239, ISSN: 1525-5050, DOI: 10.1016/J.YEBEH.2004.05.005 * the whole document * | 1-19 | |
| A | US 2019/374154 A1 (WENDLING FABRICE [FR] ET AL) 12 December 2019 (2019-12-12) * paragraphs [0068] - [0070], [0073] - [0076], [0086] * | 1-19 | |

TECHNICAL FIELDS
SEARCHED (IPC)

The present search report has been drawn up for all claims

| Place of search | Date of completion of the search | Examiner |
|---|---|---|
| Berlin | 30 November 2020 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 30 5657

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2020

| Patent document cited in search report | | Publication date | Patent family member(s) | | Publication date |
|---|---|---|---|---|---|
| US 2012296569 | A1 | 22-11-2012 | CA | 2786380 A1 | 21-07-2011 |
| | | | CN | 102906752 A | 30-01-2013 |
| | | | CN | 107468211 A | 15-12-2017 |
| | | | EP | 2526500 A2 | 28-11-2012 |
| | | | EP | 3579246 A1 | 11-12-2019 |
| | | | JP | 5951504 B2 | 13-07-2016 |
| | | | JP | 6401893 B2 | 10-10-2018 |
| | | | JP | 2013517043 A | 16-05-2013 |
| | | | JP | 2016195775 A | 24-11-2016 |
| | | | US | 2012296569 A1 | 22-11-2012 |
| | | | WO | 2011086563 A2 | 21-07-2011 |
| US 2019374154 | A1 | 12-12-2019 | CA | 3063321 A1 | 30-08-2018 |
| | | | CN | 110326054 A | 11-10-2019 |
| | | | EP | 3586339 A1 | 01-01-2020 |
| | | | FR | 3063378 A1 | 31-08-2018 |
| | | | FR | 3063379 A1 | 31-08-2018 |
| | | | JP | 2020510470 A | 09-04-2020 |
| | | | US | 2019374154 A1 | 12-12-2019 |
| | | | WO | 2018153762 A1 | 30-08-2018 |

EPO FORM P0459

For more details about this annex : see Official Journal of the European Patent Office, No. 12/82